# EUROPEAN PATENT SPECIFICATION

(11) **EP 1 749 517 B1**
(45) Date of publication and mention of the grant of the patent: **09.07.2008**
(21) Application number: 06250227.3
(22) Date of filing: 17.01.2006
(51) Int. Cl.: A61K 9/20, A61K 31/421

(54) **Stable pharmaceutical composition comprising linezolid form IV**
Stabile pharmazeutische Zusammensetzung mit Linezolid Form IV
Préparation pharmaceutique comprenant linezolide forme IV

(30) Priority: 20.07.2005 US 701438 P
(43) Date of publication of application: 07.02.2007
(73) Proprietor: Teva Pharmaceutical Industries, Inc., 49131 Petah Tiqva (IL)
(72) Inventor: Tenengauzer, Ruth, Hod Hasharon 45322 (IL); Leibovici, Minutza, Netanya 42312 (IL); Solomon, Ben-Zion, Petach Tikva 49501 (IL)
(74) Representative: Nachshen, Neil Jacob

(56) References cited:
- WO-A-01/57035
- WO-A-20/05035530
- US-A1- 2001 051 647

## Description

### FIELD OF THE INVENTION

The present invention is directed to a pharmaceutical composition containing linezolid form III that does not show more than 10% conversion to linezolid form II on storage.

The present invention is directed to a pharmaceutical composition containing linezolid form III containing not more than 20% linezolid form II.

The present invention is directed to a method of formulating linezolid to provide a pharmaceutical composition comprising linezolid wherein the linezolid is linezolid Form III containing not more than 20% of linezolid Form II and methods of inhibiting the interconversion of linezolid Form III into linezolid Form II wherein the method of manufacture is one that would normally occasion such interconversion, solid pharmaceutical compositions comprising linezolid Form III containing not more than 20% of linezolid Form II and stable solid pharmaceutical compositions comprising linezolid Form III that do not substantially convert into linezolid Form II over time, methods of treating a condition responsive to linezolid in a patient comprising administering to the patient a solid pharmaceutical composition comprising linezolid form III containing not more than 20% of linezolid Form II, and methods of treating a condition responsive to linezolid in a patient comprising administering to the patient a solid pharmaceutical composition comprising linezolid form III.

### BACKGROUND

Linezolid, chemically, N-[[(5S)-3-[3-fluoro-4-(4-morpholinyl)phenyl]-2-oxo-5-oxazolidinyl]methyl]acetamide has the chemical structure:

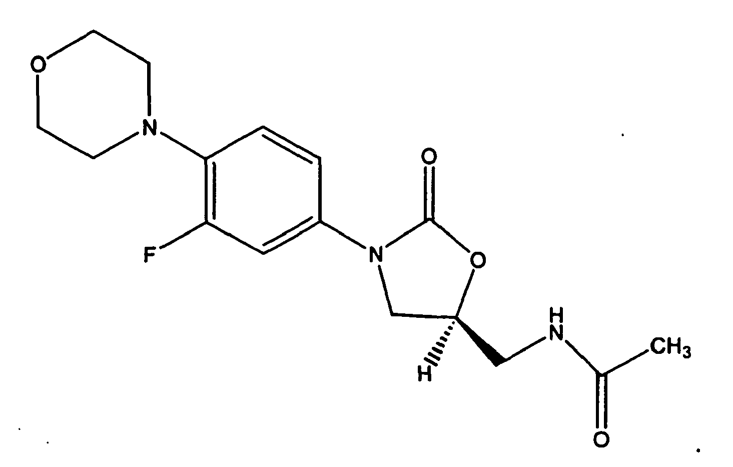

Linezolid is an anti bacterial agent.

Linezolid is known to exist in different crystal forms. Linezolid Form II is described in US patent nos. US 6444813 and US 6559305. Linezolid Form II is characterized by its X-Ray powder diffraction pattern and IR peaks. Linezolid Form II is characterized by an X-Ray powder diffraction pattern with the following peaks:

| 2-theta | relative intensity |
|---|---|
| 7.10 | 2 |
| 9.54 | 9 |
| 13.88 | 6 |
| 14.23 | 24 |
| 16.18 | 3 |
| 16.79 | 100 |
| 17.69 | 2 |
| 19.41 | 4 |
| 19.69 | 2 |
| 19.93 | 6 |
| 21.61 | 15 |
| 22.39 | 23 |
| 22.84 | 4 |
| 23.52 | 7 |
| 24.16 | 1 |
| 25.28 | 13 |
| 26.66 | 1 |
| 27.01 | 3 |
| 27.77 | 1 |

It is reported that linezolid Form II can be obtained by crystallization in a variety of solvents including water, ethyl acetate, methanol, ethanol, propanol, isopropanol, butanol, acetonitrile, acetone, methyl ethyl ketone, chloroform, toluene, and xylene.

Form III of linezolid, is described in WO 2005/035530 and is characterized by X-Ray powder diffraction pattern having peaks at 7.6, 9.6, 13.6, 14.9, 18.2, 18.9, 21.2, 22.3, 25.6, 26.9, 27.9, and 29.9 degrees 2-theta. Linezolid Form III can be obtained, for instance, by heating Form II at a temperature above about 90° C, for a period of between 2 and 12 hours.

Citation of any reference in this section of this application is not to be construed that such reference is prior art to the present application.

### SUMMARY OF THE INVENTION

The present invention is defined by claims 1 to 37 set out below.

A first aspect, the present invention is directed to a pharmaceutical composition containing polymorphically stable linezolid form III i.e. linezolid form III that does not substantially rearrange to linezolid form II and to a method of preparing a solid pharmaceutical composition comprising linezolid Form III containing not more than 20% of linezolid Form II.

A second aspect of the present invention is directed to a method of preparing a pharmaceutical composition comprising polymorphically pure linezolid Form III and to pharmaceutical compositions comprising linezolid Form III containing not more than 20% of linezolid Form II.

Definitions of the terms " polymorphically pure" and "polymorphically stable" are provided below. As defined below, the stability of polymorphically stable linezolid form III may be defined in terms of stability at 25°C at 60% relative humidity over a period of time or at 40°C and 75% relative humidity over a period of time. It is preferred that stability is defined in respect of 40°C and 75% relative humidity over a period of time, preferably 3 months. It is preferred that the polymorphically stable linezolid form III is polymorphically pure linezolid Form III.

Preferably, the polymorphically pure linezolid form III containing not more than 20% of i.e. contains less than 20% of other polymorphic forms of linezolid, more preferably less than 20% of linezolid form II. All further embodiments of the invention are described with reference to linezolid Form III containing not more than 20% of linezolid Form II, but are equally applicable to the broader definition.

In a preferred embodiment the pharmaceutical composition is solid.

In this preferred embodiment, the pharmaceutical composition is in the form of a tablet.

In this preferred embodiment, the pharmaceutical composition is in the form of a capsule.

In a further preferred embodiment the pharmaceutical composition contains povidone.

In a preferred embodiment, the pharmaceutical compositions of the first and second aspects may be prepared by techniques such as wet granulation, dry granulation and direct compression. Within each technique, preferred conditions for each embodiments described exist as discussed below.

### Wet Granulation

Thus, in one embodiment, the invention is directed to a method of preparing a solid pharmaceutical composition comprising linezolid Form III containing not more than 20% of linezolid Form II comprising wet granulating linezolid Form III containing not more than 20% of linezolid Form II with a pharmaceutically acceptable excipient using a granulation solvent selected from the group consisting of water, isopropanol, or ethanol in the presence of povidone.

In one embodiment, the linezolid Form III containing not more than 20% of linezolid Form II is admixed with an excipient that preserves Form III containing not more than 20% linezolid by wet granulating the linezolid Form III containing not more than 20% of linezolid Form II and povidone.

In one embodiment, the linezolid Form IV containing not more than 20% of linezolid Form II is admixed with povidone by wet granulating the linezolid Form III containing not more than 20% of linezolid Form II and povidone.

In one embodiment, the linezolid Form IV containing not more than 20% of linezolid Form II is admixed with povidone and at least one other excipient by wet granulating the linezolid Form III containing not more than 20% of linezolid Form II, the povidone, and at least one other excipient.

Preferably, , the solvent comprises water. In a more preferred embodiment, the solvent is water substantially free of a second solvent. In one embodiment, the solvent is water.

Alternatively, the solvent may be isopropanol or ethanol in the presence of povidone.

In these preferred embodiments, the excipient is preferably povidone, optionally with one or more other pharmaceutically acceptable excipients.

In one embodiment, the granulating solvent is isopropanol admixed with water.

In a more preferred embodiment, the granulating solvent is substantially free of ethanol.

### Dry granulation

In one embodiment, the invention is directed to a method of preparing a solid pharmaceutical composition comprising linezolid Form III containing not more than 20% of linezolid Form II comprising dry granulating linezolid Form III containing not more than 20% of linezolid Form II with a pharmaceutically acceptable excipient.

In one embodiment, the linezolid Form III containing not more than 20% of linezolid Form II is dry granulated with more than one excipients.

### Preferably, at least one excipient is povidone

In one embodiment, the linezolid Form III containing not more than 20% of linezolid Form II is admixed with povidone by dry granulation.

In one embodiment, the linezolid Form III containing not more than 20% of linezolid Form II is admixed with povidone and at least one other excipient by dry granulation.

### Direct Compression

In one embodiment, the invention is directed to a method of preparing a solid pharmaceutical composition comprising linezolid Form III containing not more than 20% of linezolid Form II comprising admixing linezolid Form III containing not more than 20% of linezolid Form II with a pharmaceutically acceptable excipient to provide a mixture and direct compressing the mixture.

In one embodiment, the linezolid Form III containing not more than 20% of linezolid Form II is admixed with more than one pharmaceutically acceptable excipient to provide a mixture and the mixture is then direct compressed.

In one embodiment, the pharmaceutical composition is obtained by admixing the linezolid Form III containing not more than 20% of linezolid Form II and an excipient that preserves Form III linezolid to provide a mixture and direct compressing the mixture.

In one embodiment, the linezolid Form III containing not more than 20% of linezolid Form II, an excipient that preserves Form III linezolid, and at least one other excipient are admixed to provide a mixture and the mixture is direct compressed.

In a preferred embodiment, povidone is an excipient.

In a preferred embodiment, the excipient that preserves Form III linezolid is povidone.

In one embodiment, the pharmaceutical composition is obtained by admixing the linezolid Form III containing not more than 20% of linezolid Form II and povidone to provide a mixture and direct compressing the mixture.

In one embodiment, the linezolid Form III containing not more than 20% of linezolid Form II, povidone, and at least one other excipient are admixed to provide a mixture and the mixture is direct compressed.

In one embodiment, the invention is directed to a method of preparing a solid pharmaceutical composition comprising linezolid Form III containing not more than 20% of linezolid Form II wherein the method comprises admixing linezolid Form III containing not more than 20% of linezolid Form II with an excipient that preserves Form III linezolid to provide a mixture.

In one embodiment, linezolid Form III containing not more than 20% of linezolid Form II is admixed with an excipient that preserves Form III linezolid and at least one other excipient to provide a mixture.

In one embodiment, linezolid Form III containing not more than 20% of linezolid Form II is admixed with more than one excipient to provide a mixture.

In one embodiment, povidone is an excipient.

In a preferred embodiment, the pharmaceutical composition is a tablet.

In an alternative preferred embodiment, the pharmaceutical composition is a capsule.

The invention further relates to a method of treating a condition responsive to linezolid in a patient comprising administering to the patient a solid pharmaceutical composition comprising linezolid form III containing not more than 20% of linezolid Form II. In one embodiment, the solid pharmaceutical composition comprising linezolid form III containing not more than 20% of linezolid Form II is prepared according to the method of the invention. In one embodiment, the condition responsive to linezolid is a bacterial infection.

The invention further relates to a method of treating a condition responsive to linezolid in a patient comprising administering to the patient a solid pharmaceutical composition comprising linezolid form III and povidone. In one embodiment, the condition responsive to linezolid is a bacterial infection.

### BRIEF DESCRIPTION OF THE DRAWINGS

FIG. 1 is an XRD diffractogram of linezolid Form III.
FIG. 2 is an XRD diffractogram of linezolid Form II.
FIG. 3 is an XRD diffractogram of the linezolid pharmaceutical composition prepared in Experiment No. 1.
FIG. 4 is an XRD diffractogram of the linezolid pharmaceutical composition prepared in Experiment No. 2.
FIG. 5 is an XRD diffractogram of the linezolid pharmaceutical composition prepared in Experiment No. 3.
FIG. 6 is an XRD diffractogram of the linezolid pharmaceutical composition prepared in Experiment No. 4.
FIG. 7 is an XRD diffractogram of the linezolid pharmaceutical composition prepared in Experiment No. 5.
FIG. 8 is an XRD diffractogram of the linezolid pharmaceutical composition prepared in Experiment No. 6.
FIG. 9 is an XRD diffractogram of the linezolid pharmaceutical composition prepared in Experiment No. 7.
FIG. 10 is an XRD diffractogram of the linezolid pharmaceutical composition prepared in Experiment No. 8.
FIG. 11 is an XRD diffractogram of the linezolid pharmaceutical composition prepared in Experiment No. 9.
FIG. 12 is an XRD diffractogram of the linezolid pharmaceutical composition prepared in Experiment No. 10.
FIG. 13 is an XRD diffractogram of the linezolid pharmaceutical composition prepared in Experiment No. 11.
FIG. 14 is an XRD diffractogram of the linezolid pharmaceutical composition prepared in Experiment No. 12.
FIG. 15 is an XRD diffractogram of the linezolid pharmaceutical composition prepared in Experiment No. 13.
FIG. 16 is an XRD diffractogram of the linezolid pharmaceutical composition prepared in Experiment No. 14.
FIG. 17 is an XRD diffiactogram of the linezolid pharmaceutical composition prepared in Experiment No. 15.
FIG. 18 is an XRD diffractogram of the linezolid pharmaceutical composition prepared in Experiment No. 16.
FIG. 19 is an XRD diffractogram of the linezolid pharmaceutical composition prepared in Experiment No. 17.
FIG. 20 is an XRD diffactogram of the linezolid pharmaceutical composition prepared in Experiment No. 18.

### DETAILED DESCRIPTION OF THE INVENTION

As used herein linezolid Form III is used to refer to the polymorphic form of linezolid disclosed in WO2005/035530

WO2005/035530 describes how this form of linezolid may be prepared for use in accordance with the present invention.

The phrase "substantially free of," as used herein, means not more than 20%. Accordingly, the phrase "linezolid Form III substantially free of linezolid Form II" means linezolid Form III containing not more than 20% of linezolid Form II. In one embodiment, the linezolid Form III contains not more than 15% of linezolid Form II. In one embodiment, the linezolid Form III contains not more than about 10% of linezolid Form II. In one embodiment, the linezolid Form III contains not more than about 5% of linezolid Form II.

The phrase "polymorphically pure compound," as that term is used herein, means a polymorph of a compound containing not more than 20% of other polymorphs of the compound and the amorphous compound. Accordingly, the phrase "polymorphically pure linezolid Form III" means linezolid Form III containing not more than 20% of other polymorphs of linezolid and amorphous linezolid. In one embodiment, the polymorphically pure linezolid Form III contains not more than 20% of other polymorphs of linezolid and amorphous linezolid. In one embodiment, the polymorphically pure linezolid Form III contains not more than 15% of other polymorphs of linezolid and amorphous linezolid. In one embodiment, the polymorphically pure linezolid Form III contains not more than 10% of other polymorphs of linezolid and amorphous linezolid. In one embodiment, the polymorphically pure linezolid Form III contains not more than 5% of other polymorphs of linezolid and amorphous linezolid.

The phrase "polymorphically stable linezolid form III," as used herein, means linezolid form III that shows not more than 10% conversion of linezolid Form III to linezolid Form II when stored at 25°C/60%RH for 3 months. In one embodiment, the linezolid form III shows not more than 10% conversion of linezolid Form III to linezolid Form II when stored at 25°C/60%RH for 6 months. In one embodiment, the linezolid form III shows not more than 30% conversion of linezolid Form III to linezolid Form II when stored at 40°C/75%RH for 3 months. In one embodiment, the linezolid form III shows not more than 25% conversion of linezolid Form III to linezolid Form II when stored at 40°C/75%RH for 3 months. In one embodiment, the linezolid form III shows not more than 20% conversion of linezolid Form II to linezolid Form II when stored at 40°C/75%RH for 3 months.

The phrase "solid dosage form" or "solid pharmaceutical composition," as used herein, means a dosage form that is a solid, *i.e.,* not a liquid, and includes, but is not limited to, tablets, capsules, sugar coated tablets, film coated tablets, enteric coated tablets, multiple compressed tablets, controlled release tablets, effervescent tablets, suppositories, and buccal and sublingual tablets (*See,* Remington The Science and Practice of Pharmacy 20th ed. ("Remington"), edited by A. Gennaro, Philadelphia College of Pharmacy and Science 2000 p. 858-856). The term "solid dosage form," as used herein, also includes suspensions of linezolid Form III. The term "suspension," as used herein means a dispersion containing finely divided insoluble material suspended in a liquid medium (*See,* Remington, p. 317).

The term "povidone," as used herein means polyvinylpyrrolidone.

The phrase "wet granulation," as used herein, means a method of manufacturing a tablet that involves adding a binder to a mixture of the active ingredient and other excipients as a solution of the binder, and is well known to one of ordinary skill in the art (*See,* Remington, p. 865-868). Typically, wet granulation involves the steps of blending an active ingredient, in this case linezolid, with one or more solid excipients such as diluents and disintegrants to provide a powdered mix; wetting the powdered mix with a granulation solvent or a solution of a binding agent in a granulation solvent to provide a damp mass; screening the damp mass; drying the damp mass to provide a dried mass; screening the dried mass to provide granules; and forming the granules into a solid dosage form such as a capsule or tablet (*See,* Remington, page 865-868).

The phrase "excipient" or pharmaceutically acceptable excipient," as used herein, means an ingredient in a pharmaceutical composition other than the active ingredient (*See,* Remington, page 860). Excipients include, but are not limited to, diluents (inert substances to increase the bulk of the pharmaceutical composition), binders (agents used to impart cohesive qualities to a powdered material), lubricants (agents that prevent adhesion of material to a die or punch, reduce inter-particle friction, facilitate ejection of a tablet from a die cavity, and/or improve the rate of flow of a powder mixture), glidants (agents that improve the flow characteristics of a powder), disintegrants (agents that facilitate the breakup or disintegration of a tablet after administration), coloring agents (agents that impart a color to a dosage form), and flavoring agents (agents that impart a flavor to a dosage form) *(See,* Remington, page 860-863). Suitable excipients include, but are not limited to, those described in Remington (*See*, Remington, page 860-863).

Water admixed with ethanol or isopropanol means a mixture of water and ethanol or isopropanol wherein the mixture contains greater than about 25% water by weight, preferably greater than about 35% water by weight, more preferably greater than about 40% water by weight, and most preferably greater than about 50% water by weight.

It has been observed that preparing a pharmaceutical composition comprising linezolid Form III and a pharmaceutically acceptable excipient by wet granulating the linezolid Form III and a pharmaceutically acceptable excipient using ethanol (not in the presence of povidone) as the granulation solvent, a common granulation solvent, the resulting pharmaceutical composition contains substantial amounts of linezolid Form II or transforms completely to form II.

By wet granulating the linezolid Form III and pharmaceutically acceptable excipient with water, isopropanol, or ethanol with the presence of povidone to form the pharmaceutical composition, the conversion of linezolid Form III to linezolid Form II is limited or even eliminated. The term "limited," as used herein, means that the amount of linezolid Form III converted to linezolid Form II is less than the amount of linezolid Form III that would be converted to linezolid Form II if a pharmaceutical composition was prepared by wet granulating the linezolid Form III and pharmaceutically acceptable excipient with ethanol (not in the presence of povidone) as the granulation solvent.

The invention further relates to a method of preparing a solid pharmaceutical composition comprising linezolid Form III containing not more than 20% of linezolid Form II wherein the method comprises wet granulating linezolid Form III containing not more than 20% of linezolid Form II with a pharmaceutically acceptable excipient using a granulation solvent that is substantially free of ethanol.

In one embodiment, povidone is a pharmaceutically acceptable excipient.

Without wishing to be bound by theory, applicants suggest that linezolid Form III is not more thermodynamically stable than linezolid Form II at room temperature. Although solid linezolid Form III is kinetically stable at room temperature, *i.e.,* it does not convert to linezolid Form II (over a time period of at least 3 months at temperatures between 25 and 40° C), we have observed that when linezolid Form III is contacted with solvents, in particular ethanol, it readily transforms to linezolid Form II, *i.e.,* the thermodynamically more stable form of linezolid at room temperature. As a consequence, pharmaceutical compositions comprising linezolid Form, III containing not more than 20% of linezolid Form II are difficult to prepare.

Thus, a more preferred aspect of the present invention relates to a method of manufacturing a pharmaceutical composition of linezolid form III containing not more than 20% of linezolid form II, that avoids the use of a liquid.

It has also been observed that the conversion of linezolid Form III to linezolid Form II can be limited by dry granulating linezolid Form III with a pharmaceutically acceptable excipient. Accordingly, the invention further relates to a method of preparing a solid pharmaceutical composition comprising linezolid Form III containing not more than 20% of linezolid Form II wherein the method comprises dry granulating linezolid Form III containing not more than 20% of linezolid Form II with a pharmaceutically acceptable excipient.

In one embodiment, the linezolid Form III substantially free of linezolid Form II is dry granulated with more than one excipients.

In one embodiment, povidone is an excipient.

The phrase "dry granulation," as used herein, means a method of manufacturing a tablet that avoids the use of a granulation solvent and the step of drying, as is required by wet granulation, and is well known to one of ordinary skill in the art (*See,* Remington, page 869). Typically, dry granulation involves the steps of weighing, mixing, slugging or compressing, dry screening, lubrication, and compression (*See*, Remington, page 869).

It has also been observed that the conversion of linezolid Form III to linezolid Form II can be limited by direct compression of linezolid Form III with a pharmaceutically acceptable excipient. Accordingly, the invention further relates to a method of preparing a solid pharmaceutical composition comprising linezolid Form III containing not more than 20% of linezolid Form II wherein the method comprises admixing linezolid Form III containing not more than 20% of linezolid Form II with a pharmaceutically acceptable excipient to provide a mixture and direct compressing the mixture.

In one embodiment, the linezolid Form III containing not more than 20% of linezolid Form II is admixed with more than one pharmaceutically acceptable excipient to provide a mixture and the mixture is then direct compressed.

In one embodiment, povidone is an excipient.

The phrase "direct compression," as used herein, means a method of manufacturing a tablet by compressing tablets directly from powdered material without modifying the nature of the material itself, and is well known to one of ordinary skill in the art (*See,* Remington, page 869-870).

It has also been observed that specific excipients, called "excipients that preserve Form III linezolid" limit the conversion of linezolid Form III to linezolid Form II, even when the pharmaceutical composition is prepared by wet granulation with ethanol as the granulation solvent. Accordingly, the invention further relates to a method of preparing a solid pharmaceutical composition comprising linezolid Form III containing not more than 20% of linezolid Form II wherein the method comprises admixing linezolid Form III containing not more than 20% of linezolid Form II with an excipient that preserves Form III linezolid. The term "limit," as used herein, means that the amount of linezolid Form III converted to linezolid Form II is less than the amount of linezolid Form III that would be converted to linezolid Form II if a pharmaceutical composition was prepared by in the absence of excipients that preserve Form III linezolid.

In one embodiment, linezolid Form. III containing not more than 20% of linezolid Form II is admixed with an excipient that preserves Form III linezolid and at least one other excipient to provide a mixture.

In one embodiment, the invention relates to a method of preparing a solid pharmaceutical composition comprising linezolid Form III containing not more than 20% of linezolid Form II wherein the method comprises admixing linezolid Form III containing not more than 20% of linezolid Form II with an excipient that preserves Form III linezolid to provide a mixture and wet granulating the mixture using a solvent comprising ethanol as the granulating solvent. In one embodiment, the granulating solvent is ethanol

An example of an excipient that preserves Form III linezolid is povidone. Accordingly, the invention further relates to a method of preparing a solid pharmaceutical composition comprising linezolid Form III containing not more than 20% of linezolid Form II wherein the method comprises admixing linezolid Form III containing not more than 20% linezolid Form II with povidone.

In one embodiment, the method involves admixing linezolid Form III containing not more than 20% of linezolid Form II, povidone, and at least one other pharmaceutically acceptable excipient.

In one embodiment, the linezolid Form III containing not more than 20% of linezolid Form II is admixed with povidone by wet granulating the linezolid Form III containing not more than 20% of linezolid Form II and povidone. In one embodiment, the granulating solvent is ethanol. In one embodiment, the granulating solvent is ethanol admixed with water. In one embodiment, the granulating solvent is isopropanol. In one embodiment, the granulating solvent is isopropanol admixed with water. In one embodiment, the granulating solvent is water.

In one embodiment, linezolid Form III containing not more than 20% of linezolid Form II, povidone, and at least one other pharmaceutically acceptable excipient are admixed by wet granulation.

In one embodiment, the linezolid Form III containing not more than 20% of linezolid Form II is admixed with povidone by dry granulation.

In one embodiment, the linezolid Form III containing not more than 20% of linezolid Form II is admixed with povidone and at least other excipient by dry granulation.

In one embodiment, the pharmaceutical composition is obtained by admixing the linezolid Form III containing not more than 20% of linezolid Form II and povidone to provide a mixture and direct compressing the mixture.

In one embodiment, the linezolid Form III containing not more than 20% of linezolid Form II is admixed with povidone and at least other excipient to provide a mixture and direct compressing the mixture.

The invention further relates to a solid pharmaceutical composition comprising linezolid Form III containing not more than 20% of linezolid Form II.

The invention further relates to a solid pharmaceutical composition comprising linezolid Form III containing not more than 20% of linezolid Form II, and povidone.

In one embodiment, the pharmaceutical composition is a tablet. In one embodiment, the pharmaceutical composition is a capsule. The solid pharmaceutical compositions are made using methods well known to those skilled in the art (*See,* Remington, p. 858-893).

The invention further relates to the use of linezolid form III in the manufacture of a medicament for treating a condition responsive to linezolid in a patient comprising administering to the patient a solid pharmaceutical composition comprising linezolid form III containing not more than 20% of linezolid Form II. In one embodiment, the solid pharmaceutical composition comprising linezolid form III containing not more than 20% of linezolid Form II is prepared according to the method of the invention. In one embodiment, the condition responsive to linezolid is a bacterial infection.

The invention further relates to the use of linezolid Form III in the manufacture of a medicament for treating a condition responsive to linezolid in a patient comprising administering to the patient a solid pharmaceutical composition comprising linezolid form III and povidone. In one embodiment, the condition responsive to linezolid is a bacterial infection.

In each of the above-described aspects and embodiments of the invention, the composition is preferably free containing not more than 20% of sugar alcohols such as mannitol.

### Linezolid Form III and Form II

The characteristic peaks in the XRD diffractogram of linezolid Form II are found at about 7.1 ± 0.2, 9.6 ± 0.2, 14.2 ± 0.2, 16.9 ± 0.2, 21.7 ± 0.2, 22.5 ± 0.2, and 23.6 ± 0.2 degrees 2-theta.

The characteristic peaks in the XRD diffractogram of linezolid Form III are found at about 7.4 ± 0.2, 9.4 ± 0.2, 13.6 ± 0.2, 14.8 ± 0.2, 15.2 ± 0.2, 15.4 ± 0.2, 16.3 ± 0.2, 16.9 ± 0.2,18.0 ± 0.2, 18.5 ± 0.2, 18.8 ± 0.2, 21.0 ± 0.2, 22.3 ± 0.2, and 29.7 + 0.2 degrees 2-theta.

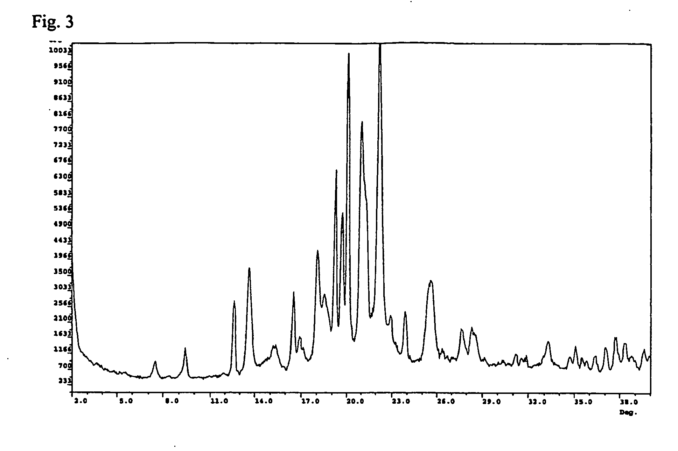

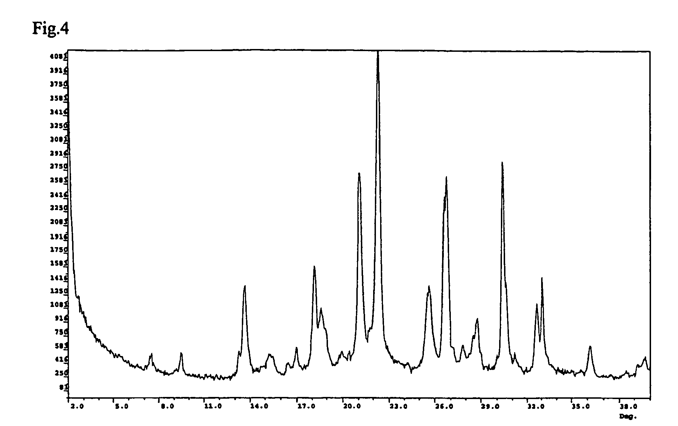

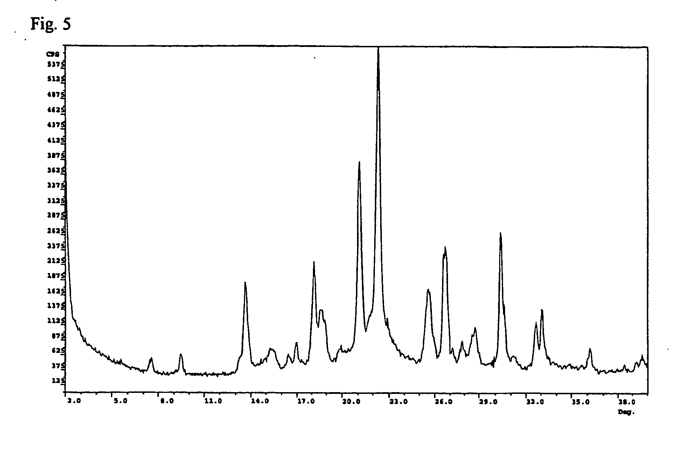

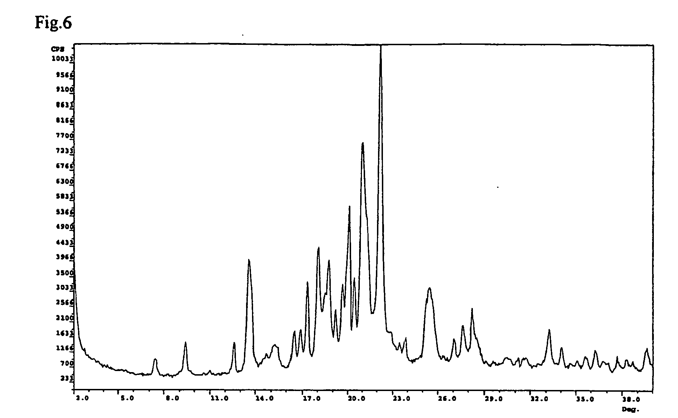

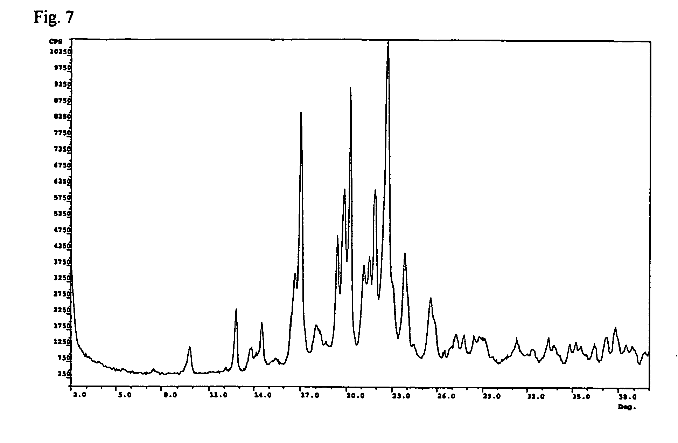

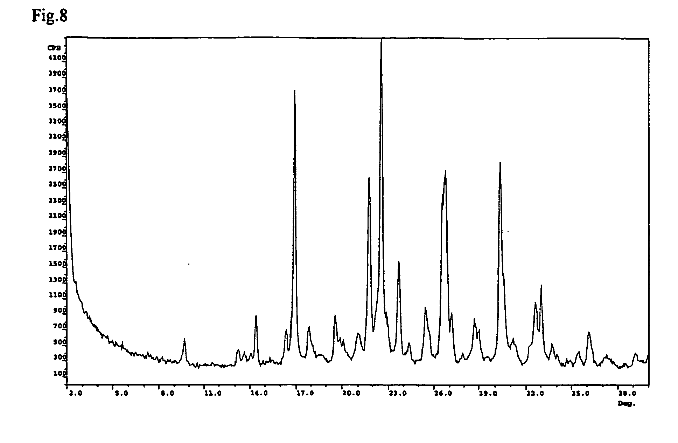

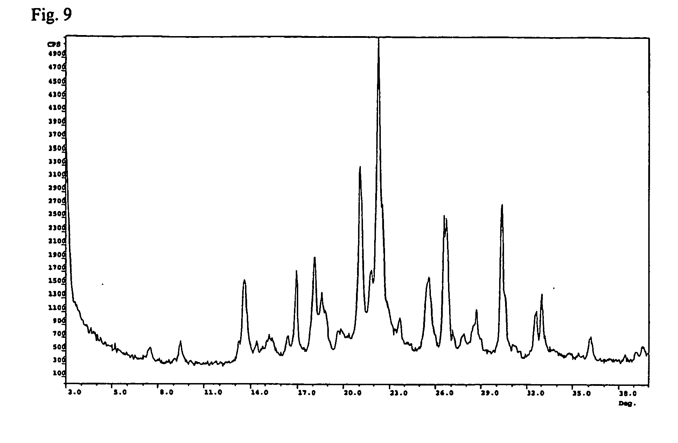

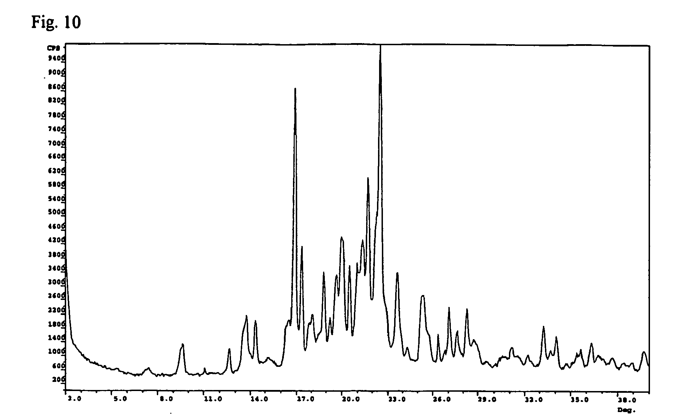

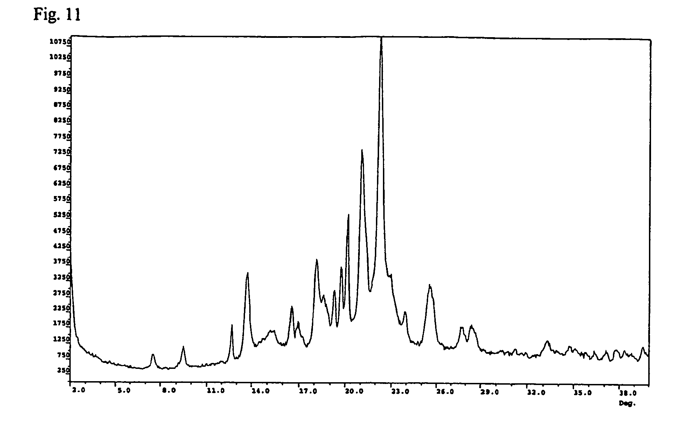

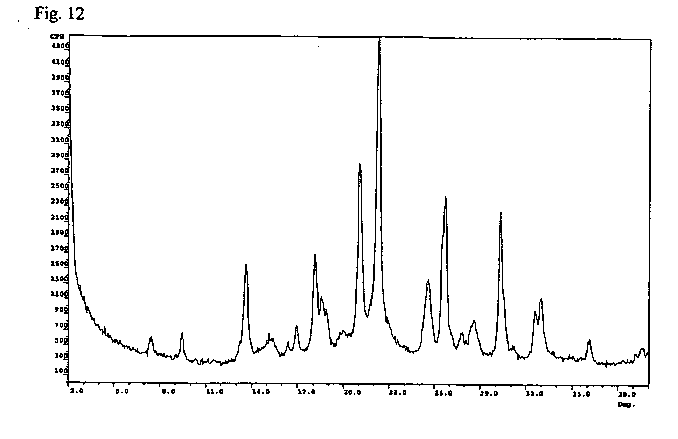

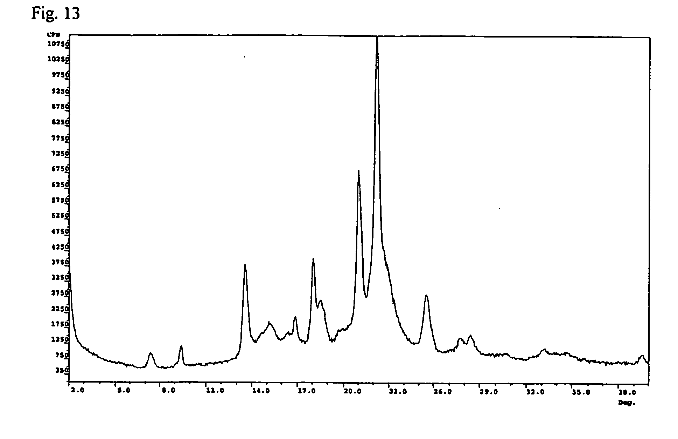

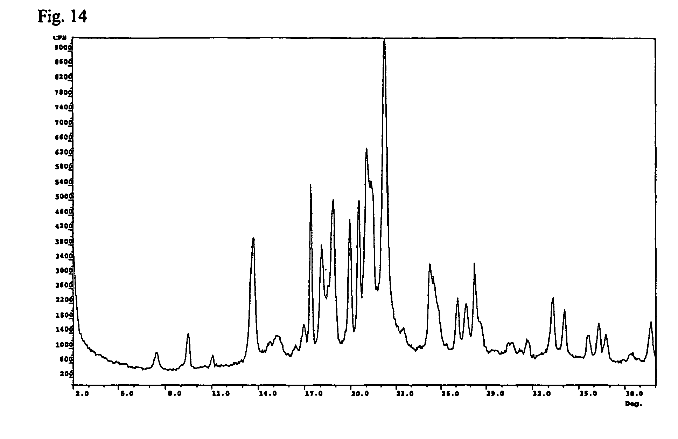

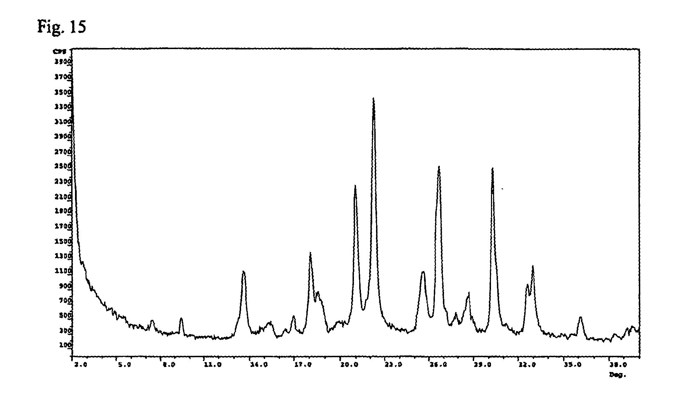

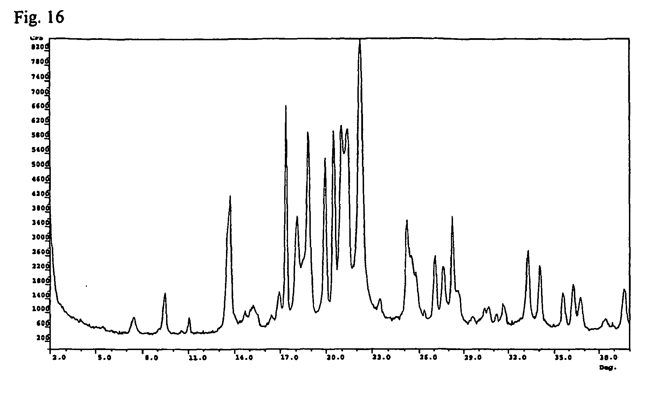

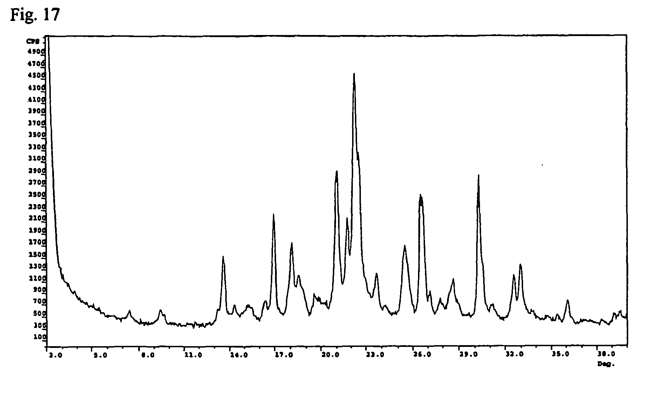

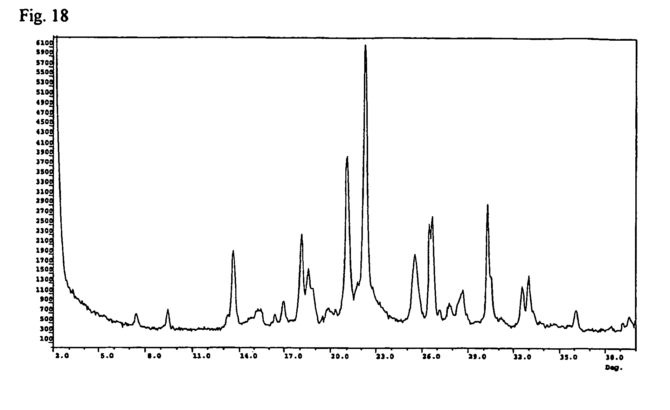

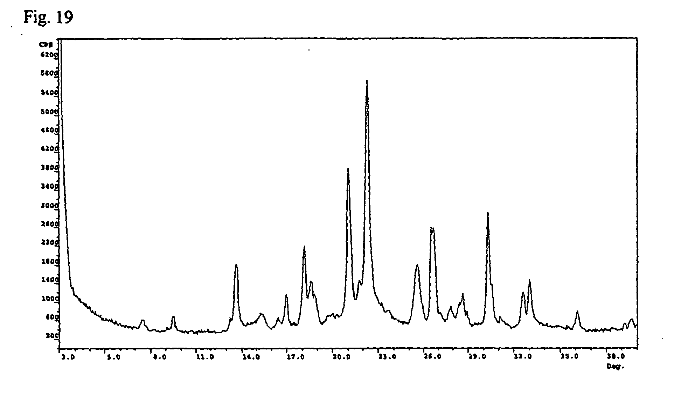

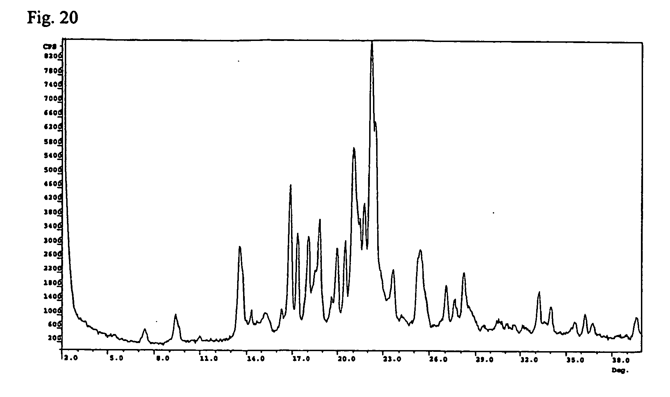

FIGS. 3-20 depict the XRD diffractogram of the pharmaceutical compositions prepared above. By knowing the characteristic peaks in the XRD of linezolid Form II and linezolid Form III it is possible to determine the crystal Form of the linezolid in each pharmaceutical composition. The XRD diffractogram of the pharmaceutical compositions includes peaks from the linezolid and the excipients included in the pharmaceutical formulation. By knowing which peaks in the XRD diffractogram of the pharmaceutical composition are due to the excipients, it is possible to identify the peaks that are due to linezolid and, therefore, to identify whether the linezolid in the pharmaceutical composition is linezolid Form III or linezolid Form II. Although, it may not be possible in an XRD diffractogram of a pharmaceutical composition to identify every one of the characteristic peaks of linezolid Form II or linezolid Form III identified above (for example, because peaks from an excipient may interfere with or overlap with peaks from linezolid), a sufficient number of peaks corresponding to linezolid Form III or linezolid Form II can be identified for one of ordinary skill in the art to characterize the linezolid in the pharmaceutical composition as linezolid Form III or linezolid Form II. Typically, the occurrence of peaks in the XRD diffractogram of a pharmaceutical composition at 7.4, 9.4, 13.6, 18.0, 21.0 degrees 2-theta (± 0.2) is sufficient to show that the linezolid present in the pharmaceutical composition is linezolid Form III Similarly, the occurrence of peaks in the XRD diffractogram of a pharmaceutical composition at 14.2,, 21.7, 23.6 degrees 2-theta (± 0.2) is sufficient to show that the linezolid present in the pharmaceutical composition in addition to form III is linezolid Form II. The choice of the specific peaks, however, may vary if the excipients used will be different.

Techniques other than X-ray powder diffraction, well known to those of ordinary skill in the art, can also be used to identify and quantify polymorphs in pharmaceutical compositions such as tablets. Representative other methods include, but are not limited to, solid-state NMR and infra-red spectroscopy.

### EXAMPLES

Various pharmaceutical compositions, in the form of a tablet, containing 300 mg of linezolid Form III, were prepared by wet granulation, dry granulation, or direct compression.

### Examples 1-4

### Method - Wet granulation.

### Granulation Solution -Purified Water

### Procedure:

1. Mix together the components of part I.
2. Add granulation solution (Purified Water) to the mix from step 1 to form a granulate.
3. Dry and mill the granulate from step 2.
4. Add components of part II and III to the milled granulate from step 3 and mix to get a final blend.
5. Compress the final blend into tablets.

**Table 1**

| **Ingredients** | **Example 1 mg/tab** | **Example 2 mg/tab** | **Example 3 mg/tab** | **Example 4 mg/tab** |
|---|---|---|---|---|
| **Part I** | | | | |
| Linezolid | 300.0 | 300.0 | 300.0 | 300.0 |
| Starch NF | 100.0 | 100.0 | 100.0 | 100.0 |
| Lactose Monohydrate NF | 100.0 | ----- | ----- | ----- |
| Mannitol | ----- | ----- | ----- | 100.0 |
| Cal. Phosphate Dibasic Anhyd. USP | ----- | 100.0 | 100.0 | ----- |
| Povidone USP (PVP K-30) | ---- | ----- | 30.0 | ----- |
| Hydroxy Methyl Cellulose NF | 16.0 | 16.0 | ----- | 16.0 |
| Croscarmellose Sodium NF | ----- | ----- | 24.0 | ----- |
| Crospovidone USP | 24.0 | 24.0 | ----- | 24.0 |

| **Part II** | | | | |
|---|---|---|---|---|
| Lactose Spray Dried | 54.0 | ----- | ----- | 54.0 |
| Microcrystalline Cellulose NF | ----- | ----- | 40.0 | ----- |
| A-Tab (Cal. Phosphate Dibasic Anhyd. USP) | ----- | 54.0 | ----- | ----- |

| **Part III** | | | | |
|---|---|---|---|---|
| Magnesium Stearate NF | 6.0 | 6.0 | 6.0 | 6.0 |
| Total | 600.0 | 600.0 | 600.0 | 600.0 |

### Examples 5-8

### Method -- Wet granulation

### Granulation Solution - Ethanol

### Procedure:

1. Mix together the components of part I.
2. Add granulation solution (Ethanol) to the mix from step 1 to form a granulate.
3. Dry and mill the granulate from step 2.
4. Add components of part II and III to the milled granulate from step 3 and mix to get a final blend.
5. Compress the final blend into tablets.

**Table 2**

| **Ingredients** | **Example 5 mg/tab** | **Example 6 mg/tab** | **Example 7 mg/tab** | **Example 8 mg/tab** |
|---|---|---|---|---|
| **Part I** | | | | |
| Linezolid | 300.0 | 300.0 | 300.0 | 300.0 |
| Starch NF | 100.0 | 100.0 | 100.0 | 100.0 |
| Lactose Monohydrate NF | 100.0 | ----- | ----- | ----- |
| Cal. Phosphate Dibasic Anhyd. USP | ----- | 100.0 | 100.0 | ----- |
| Mannitol | ----- | ----- | ----- | 100.0 |
| Povidone USP (PVP K-30) | ---- | ----- | 30.0 | ----- |
| Hydroxy Methyl Cellulose NF | 16.0 | 16.0 | ----- | 16.0 |
| Croscarmellose Sodium NF | ---- | ----- | 24.0 | ----- |
| Crospovidone USP | 24.0 | 24.0 | ----- | 24.0 |

| **Part II** | | | | |
|---|---|---|---|---|
| Lactose Spray Dried | 54.0 | ----- | ----- | 54.0 |
| Microcrystalline Cellulose NF | ----- | ---- | 40.0 | ----- |
| A-Tab (Cal. Phosphate Dibasic Anhyd. USP) | ---- | 54.0 | ----- | ---- |

| **Part III** | | | | |
|---|---|---|---|---|
| Magnesium Stearate NF | 6.0 | 6.0 | 6.0 | 6.0 |
| Total | 600.0 | 600.0 | 600.0 | 600.0 |

### Examples 9-12

### Method - Direct Compression

### Procedure:

1. Mix together the components of part I.
2. Add Magnesium Stearate of Part II to the mix from step 1 and mix to get a final mix.
3. Compress the final mix from step 2 into tablets.

**Table 3**

| **Ingredients** | **Example 9 mg/tab** | **Example 10 mg/tab** | **Example 11 mg/tab** | **Example 12 mg/tab** |
|---|---|---|---|---|
| **Part I** | | | | |
| Linezolid | 300.0 | 300.0 | 300.0 | 300.0 |
| Starch 1500 | 100.0 | 100.0 | 100.0 | 100.0 |
| Mannitol | ----- | ----- | ----- | 200.0 |
| Povidone USP (PVP K-30) | 30.0 | ----- | 30.0 | 30.0 |
| Hydroxy Methyl Cellulose NF | ---- | 30.0 | ----- | ----- |
| Croscarmellose Sodium NF | ---- | 24.0 | ----- | ----- |
| Crospovidone USP | 24.0 | ----- | 24.0 | 24.0 |
| Lactose Spray Dried | 100.0 | ---- | ----- | ----- |
| Microcrystalline Cellulose NF | 140.0 | 40.0 | 240.0 | 40.0 |
| A-Tab (Cal. Phosphate Dibasic Anhyd. USP) | ----- | 200.0 | ----- | ----- |

| **Part II** | | | | |
|---|---|---|---|---|
| Magnesium Stearate NF | 6.0 | 6.0 | 6.0 | 6.0 |
| Total | 700.0 | 700.0 | 700.0 | 700.0 |

### Examples 13-14

### Method - Dry Granulation.

### Procedure:

1. Mix together the components of Part I and II.
2. Compress the mix from step 1 into tablets (slugs).
3. Mill the tablets from step 2, add components of part III and IV, and mix well to get a final blend.
4. Compress the final blend from step 3 into tablets.

**Table 4**

| **Ingredients** | **Example 13 mg/tab** | **Example 14 mg/tab** |
|---|---|---|
| **Part I** | | |
| Linezolid | 300.0 | 300.0 |
| Starch NF | 100.0 | 100.0 |
| Mannitol | ----- | 100.0 |
| Povidone USP (PVP K-30) | 30.0 | ----- |
| Hydroxy Methyl Cellulose NF | ---- | 16.0 |
| A-Tab (Cal. Phosphate Dibasic Anhyd. USP) | 100.0 | ----- |
| Crospovidone USP | 24.0 | 24.0 |

| **Part II** | | |
|---|---|---|
| Magnesium Stearate NF | 5.0 | 5.0 |

| **Part III** | | |
|---|---|---|
| Mannitol | ----- | 150 |
| A-Tab (Cal. Phosphate Dibasic Anhyd. USP) | 136.0 | ----- |

| **Part IV** | | |
|---|---|---|
| Magnesium Stearate NF | 5.0 | 5.0 |
| Total | 700.0 | 700.0 |

### Examples 15-18

### Method - Wet granulation.

### Granulation Solution - See table below

### Procedure:

1. Mix together the components of part I.
2. Add the granulation solution and mix to form a granulate.
3. Dry wet granulate from step 2 and mill.
4. Add components of part II and III to the milled granulate from step 3 and mix to get a final blend.
5. Compress the final blend from step 4 into tablets.

**Table 5**

| **Ingredients** | **Example 15 mg/tab** | **Example 16 mg/tab** | **Example 17 mg/tab** | **Example 18 mg/tab** |
|---|---|---|---|---|
| **Part I** | | | | |
| Linezolid | 300.0 | 300.0 | 300.0 | 300.0 |
| Starch NF | 100.0 | 100.0 | 100.0 | 100.0 |
| Cal. Phosphate Dibasic Anhyd. USP | 100.0 | 100.0 | 100.0 | ----- |
| Mannitol | ----- | ----- | ----- | 100.0 |
| Povidone USP (PVP K-30) | 30.0 | 30.0 | 30.0 | 30.0 |
| Hydroxy Methyl Cellulose NF | ----- | ----- | ----- | ----- |
| Croscarmellose Sodium NF | ---- | ----- | ----- | ----- |
| Crospovidone USP | 24.0 | 24.0 | 24.0 | 24.0 |
| **Granulation Solution** | Ethanol/Purified Water 1:1 | iso-propyl alcohol | PVP K-30 in Alcohol | PVP K-30 in Alcohol/Purified Water |

| **Part II** | | | | |
|---|---|---|---|---|
| Microcrystalline Cellulose NF | 40.0 | 40.0 | 40,0 | 40.0 |

| **Part III** | | | | |
|---|---|---|---|---|
| Magnesium Stearate NF | 6.0 | 6.0 | 6.0 | 6.0 |
| Total | 600.0 | 600.0 | 600.0 | 600.0 |

The polymorphic content of each of the pharmaceutical compositions was determined by x-ray powder diffraction ("XRD"). XRD diffractograms were obtained using a Scintag X-Ray powder diffractometer model X'TRA with a Cu tube and a solid state detector. For sampling a round standard aluminum sample holder with a round zero background quartz plate was used. The following scanning parameters were used: Regular scan, *i.e.,* 2-40 degrees 2θ, continuous scan, rate 3.00 degree/min.

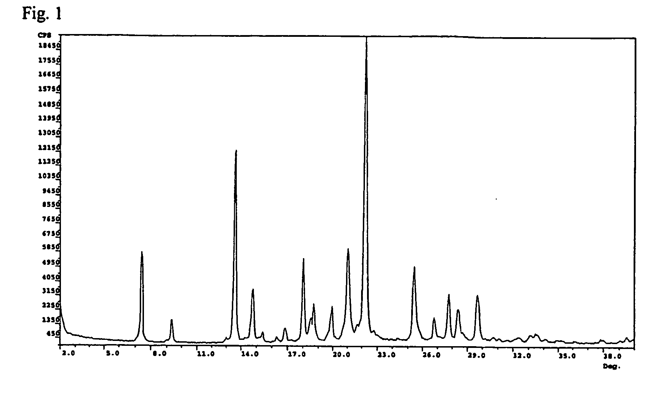

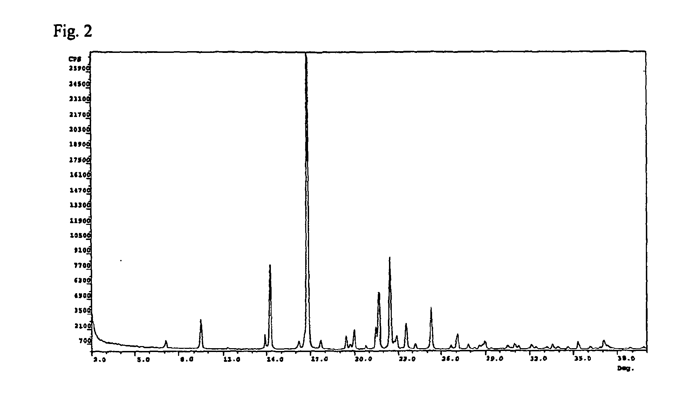

FIG. 1 depicts the XRD diffractogram of linezolid Form III and FIG. 2 depicts the XRD diffractogram of linezolid Form II.

The characteristic peaks in the XRD diffractogram of linezolid Form II are found at about 7.1 ± 0.2, 9.6 ± 0.2, 14.2 ± 0.2, 16.9 ± 0.2, 21.7 ± 0.2, 22.5 ± 0.2, and 23.6 ± 0.2 degrees 2-theta.

The characteristic peaks in the XRD diffractogram of linezolid Form III are found at about 7.4 ± 0.2, 9.4 ± 0.2, 13.6 ± 0.2, 14.8 ± 0.2, 15.2 ± 0.2, 1 5.4 ± 0.2, 16.3 ± 0.2, 16.9 ± 0.2, 18.0 ± 0.2, 18.5 ± 0.2, 18.8 ± 0.2, 21.0 ± 0.2, 22.3 ± 0.2, and 29.7 ± 0.2 degrees 2-theta.

FIGS. 3-20 depict the XRD diffractogram of the pharmaceutical compositions prepared above. By knowing the characteristic peaks in the XRD of linezolid Form II and linezolid Form III it is possible to determine the crystal Form of the linezolid in each pharmaceutical composition. The XRD diffractogram of the pharmaceutical compositions includes peaks from the linezolid and the excipients included in the pharmaceutical formulation. By knowing which peaks in the XRD diffractogram of the pharmaceutical composition are due to the excipients, it is possible to identify the peaks that are due to linezolid and, therefore, to identify whether the linezolid in the pharmaceutical composition is linezolid Form III or linezolid Form II. Although, it may not be possible in an XRD diffractogram of a pharmaceutical composition to identify every one of the characteristic peaks of linezolid Form II or linezolid Form III identified above (for example, because peaks from an excipient may interfere with or overlap with peaks from linezolid), a sufficient number of peaks corresponding to linezolid Form III or linezolid Form II can be identified for one of ordinary skill in the art to characterize the linezolid in the pharmaceutical composition as linezolid Form III or linezolid Form II. Typically, the occurrence of peaks in the XRD diffractogram of a pharmaceutical composition at 7.4, 9.4, 13.6, 18.0, 21.0 degrees 2-theta (± 0.2) is sufficient to show that the linezolid present in the pharmaceutical composition is linezolid Form III Similarly, the occurrence of peaks in the XRD diffractogram of a pharmaceutical composition at 14.2,, 21.7, 23.6 degrees 2-theta (± 0.2) is sufficient to show that the linezolid present in the pharmaceutical composition in addition to form III is linezolid Form II. The choice of the specific peaks, however, may vary if the excipients used will be different.

Techniques other than X-ray powder diffraction, well known to those of ordinary skill in the art, can also be used to identify and quantify polymorphs in pharmaceutical compositions such as tablets. Representative other methods include, but are not limited to, solid-state NMR and infra-red spectroscopy.

Table 6 summarizes the crystal Form of the linezolid in each of the pharmaceutical compositions upon manufacture.

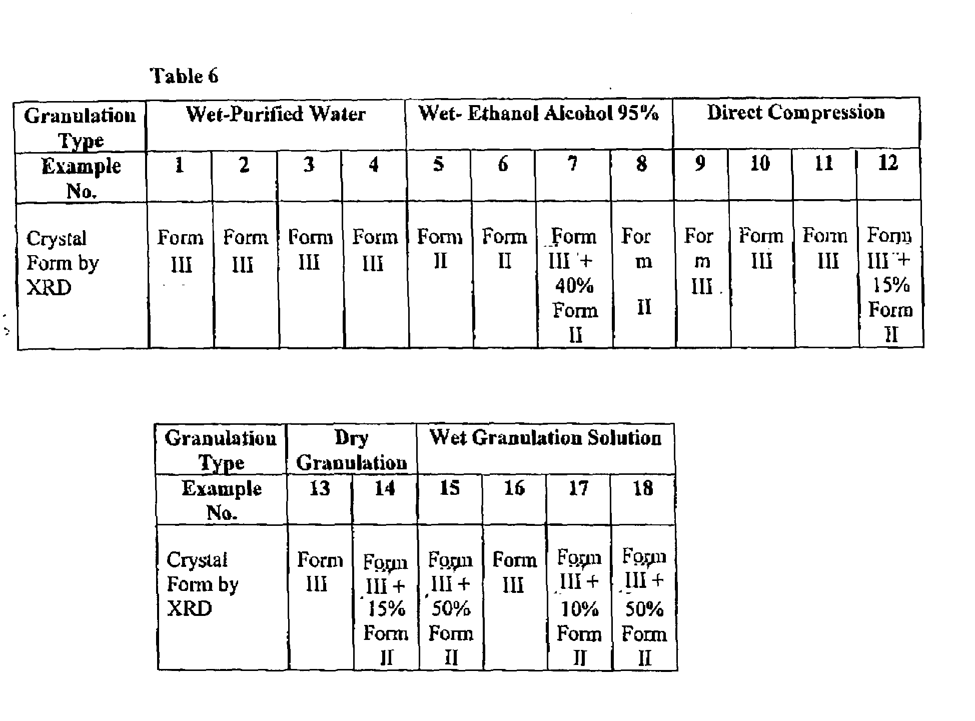

Table 7 summarizes the peaks of the active ingredient detected in the X-Ray diffractograms of the tablets.

**Table 7**

| **Number of experiment** | **XRD peaks of the active ingredient detected in the diffractograms of the tablets** |
|---|---|
| 1 | Peaks of Form III at about 7.4, 9.4, 13.6, 18.1, 18.5, 22.2 degrees 2-theta |
| 2 | Peaks of Form III at about 7.4, 9.4, 13.5, 15.2-15.5 (broad), 16.3, 16.9, 18.0, 18.5, 18.7 21.1, 22.3 degrees 2-theta |
| 3 | Peaks of Form III at about 7.5, 9.5, 13.6, 15.3-15.5 (broad), 16.4, 17.0, 18.1, 18.6,18.9 (shoulder), 21.1,22.4 degrees 2-theta |
| 4 | Peaks of Form III at about 7.4, 9.4, 13.6, 14.7, 15.1-15.4 (broad), 16.9, 18.0, 18.5,21.0, 22.3 degrees 2-theta |
| 5 | Peaks of Form II at about 9.5, 14.2, 16.9, 22.4, 23.6, 21.6, 25.2 degrees 2-theta |
| 6 | Peaks of Form II at about 9.5, 14.2, 16,2, 16.8, 19.4, 21.7, 22.4,23.6, 25,3 degrees 2-theta |
| 7 | Peaks of Form III at about 7.4, 9.4, 13.6, 18.1, 18.5, 18.8 (shoulder), 21.0, 22.3 and peak of Form II at about 14.2, 23.6 degrees 2-theta. |
| 8 | Peaks of Form II at about 9,6, 14.3, 16.9, 21.8, 22.5, 23.7 degrees 2-theta. |
| 9 | Peaks of Form III at about 7.5, 9.5, 13.6, 16.9, 18.1, 18.5, 21.0, 22.3 degrees 2-theta |
| 10 | Peaks of Form III at about 7.5, 9.5, 13.6, 16.4, 16.9, 18.1, 18.5, 18.8, 21.1, 22.3, 25.5degrees 2-theta |
| 11 | Peaks of Form III at about 7.4, 9.4, 13.6, 15.1-15.4 (broad), 16.4, 16.9, 18.0, 18.5, 21.0, 22.2 degrees 2-theta |
| 12 | Peaks of Form III at about 7.4, 9.5, 13.7, 16.4, 16.9, 18.2, 18.8, 25.3, 25.5 degrees 2-theta and peak of Form II at about 23.6 degrees 2-theta. |
| 13 | Peaks of Form II at about7.4, 9.4, 13,6, 15.2-15.5 (broad), 16.9,18.1, 18.5, 21.0, 22.2 degrees 2-theta |
| 14 | Peaks of Form III at about 7.5, 9.5, 14.7, 15.1-15.5 (broad), 16.4, 16,9, 18.1, 18.5, 18.9 (shoulder), 21.1, 22.3 degrees 2-theta and peak of Form II at about 23.5 degrees 2-theta. |
| 15 | Peaks of Form III at about7.4, 9.4,13.5,15.2-15.5 (broad), 16.3, 16.9,18.1, 18.5, 21.1, 22.2 degrees 2-theta and peak of Form II at about 9.7, 14.4, 23.6 degrees 2-theta. |
| 16 | Peaks of Form III at about 7.4, 9.4, 13.6, 15.1-15.5 (broad), 16.3, 16.9, 18.1, 18.5, 21.1, 22.3 degrees 2-theta. |
| 17 | Peaks of Form III at about 7.5, 9.5, 13.6, 15.2-15.5 (broad), 16.4, 17.0, 18.2, 18.6,21.1, 22.3 degrees 2-theta and peak of Form II at about 23.6 degrees 2-theta. |
| 18 | Peaks of Form III at about 7.3, 9.4, 13.5, 15.3-15.5 (broad), 18.0, 18.5, 21.0, 22.2 degrees 2-theta and peak of Form II at about 14.2, 21.7, 23.4 degrees 2-theta. |

All the formulations, disregarding the polymorphic composition, were extremely stable at 25°C/60%RH for 6 or 3 months, in the sense that NMT 10% conversion of Form III to Form II occurred. (See Table 8).

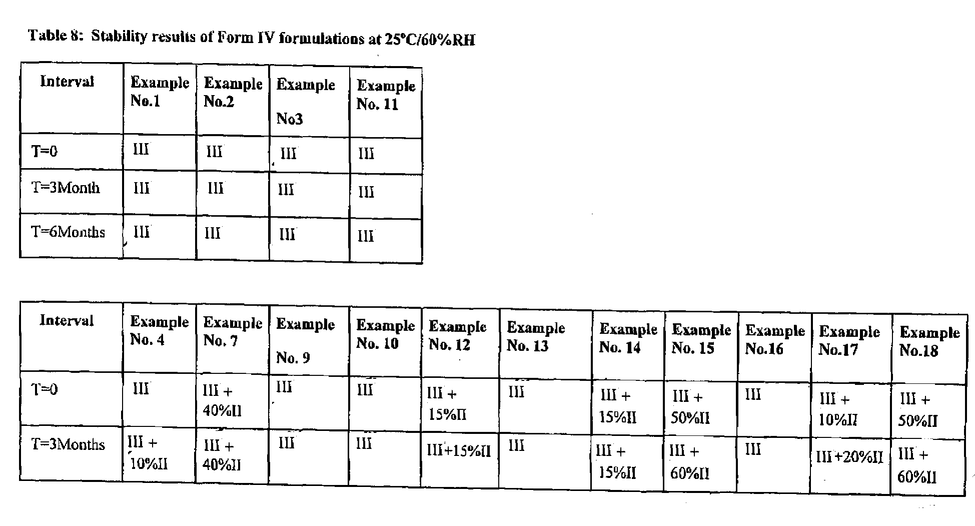

It was observed that all the solid compositions prepared according to the methods of the invention by wet granulating with water, isopropanol, or ethanol in the presence of povidone are stable at 40°C/75%RH (*See* tables 9 and 10), according to the following criteria:
Not more than 30% conversion of linezolid Form III to linezolid Form II after 3 months (ex. 4, 7, 9, 11, 15, 17, 18);
Not more than 25% conversion of linezolid Form III to linezolid Form II after 2 months (ex. 4, 7, 9, 11, 12, 15, 17, 18);
Not more than 20% conversion of linezolid Form III to linezolid Form II after 1 month (ex., 7, 9, 10, 11, 15, 17, 18).

A pharmaceutical composition comprising linezolid Form III wherein there is not more than 30% conversion of linezolid Form III to linezolid Form II after 3 months when the composition is maintained at 40°C/75%RH is a composition comprising "polymorphically stable linezolid Form III."

**Table 9: Stability results of Form IV formation at 40°C/75%RH (no detectable conversion to Form II)**

| Interval | Example No.1 | Example No.2 | Example No.3 | Example No.16 |
|---|---|---|---|---|
| T=0 | III | III | III | III |
| T=1Month | III | III | III | III |
| T=2Months | III | III | III | III |
| T=3Months | III | III | III | III |

The results in Table 9 show that there is no detectable conversion of Form III to Form II in the compositions of Examples 1, 2, 3, and 16.

**Table 10: Stability results of Form III formulations at 40°C/75%RH**

| Interval | Example No.4 | Example No.7 | Example. No.9 | Example No. 10 | Example No. 11 | Example No. 12 | Example No. 13 | Example No.15 | Example No.17 | Example No.18 |
|---|---|---|---|---|---|---|---|---|---|---|
| T=0 | III | III+40%II | III | III | III | III+15%II | III | III+50%II | III+10%II | III+60% II |
| T=1Month | III | III+50%II | III | III | III | III+15%II | III+50%II | III+60%II | III+20%II | III+60%II |
| T=2Month | III+25%II | III+55%II | III+20%II | III+40%II | III+10%II | III+40%II | III+70%II | III+60%II | III+25%II | III60%II |
| T=3Month | III+30%II | III+60%II | III+35%II | III+65%II | III+10%II | III+50%II | III+80%II | III+70%II | III+35%II | III+65%II |

It is believed that in the formulations of wet granulation with water or isopropanol, form III is preserved with time disregarding the excipients present.(Example No. 1, 2, 3, 16)

It is also believed that the formulations of Example Number 9, 11, 12, 13, 15, 17, 18, *i.e.,* where the formulation is dry or other solvents were used instead of or in combination with water, the presence of PVP enhances the stability toward polymorphic transformation.

The results of the above described experiments demonstrate the following:

Wet granulation with water or isopropanol is better than granulation with ethanol to provide a formulation with Form II containing not more than 20% of Form II.

Wet granulation with ethanol containing povidone is better than granulation with ethanol in the absence of povidone containing not more than 20%.

Povidone inhibits the conversion of Form III to Form II even under conditions were the conversion of Form III to Form II is likely (e.g., in the presence of ethanol). Formulating a pharmaceutical containing povidone by adding the povidone to the composition as a solution of povidone is better than admixing solid povidone with other excipients.

Wet granulation advantageously provides a formulation with Form III substantially free of Form II.

It is advantageous to avoid using sugar alcohols, in particular mannitol, as an excipient. Even in dry granulation methods it is advantageous to avoid using sugar alcohols, in particular mannitol, as an excipient.

The present invention is not to be limited in scope by the specific embodiments disclosed in the examples which are intended as illustrations of a few aspects of the invention and any embodiments that are functionally equivalent are within the scope of this invention. Indeed, various modifications of the invention in addition to those shown and described herein will become apparent to those skilled in the art and are intended to fall within the scope of the appended claims.

## Claims

1. A pharmaceutical composition comprising polymorphically stable linezolid Form III and a pharmaceutically acceptable excipient **characterized in that** the pharmaceutical composition shows not more than 10% conversion of linezolid Form III to linezolid Form II when stored at 25°C/60%RH for 3 months., said composition being prepared by a method that avoids liquids or wet granulation using a granulation solvent selected from the group consisting of water, isopropanol, ethanol in the presence of povidone and water admixed with isopropanol or water admixed with ethanol in the presence of povidone.

2. The pharmaceutical composition of claim 1, wherein the excipient is povidone.

3. The pharmaceutical composition of claim 1, wherein the pharmaceutical composition is free of mannitol.

4. The pharmaceutical composition of claim 1, wherein the pharmaceutical composition is a solid.

5. The pharmaceutical composition of claim 4, wherein the pharmaceutical composition is in the form of a tablet or a capsule.

6. The pharmaceutical composition of claim 1, wherein the pharmaceutical composition shows less than 30 percent conversion of linezolid Form III to linezolid Form II when the pharmaceutical composition is maintained at 40° C and 75% relative humidity for 3 months.

7. The pharmaceutical composition of claim 1, wherein the pharmaceutical composition shows less than 25 percent conversion of linezolid Form III to linezolid Form II when the pharmaceutical composition is maintained at 40° C and 75% relative humidity for 2 months.

8. The pharmaceutical composition of claim 1, wherein the pharmaceutical composition shows less than 20 percent conversion of linezolid Form III to linezolid Form II when the pharmaceutical composition is maintained at 40° C and 75% relative humidity for 1 months.

9. A pharmaceutical composition according to any preceding claim, where the polymorphically stable linezolid form III is polymorphically pure linezolid Form III and a pharmaceutically acceptable excipient.

10. A pharmaceutical composition according to any preceding claim, where the polymorphically pure linezolid form III contains less than 20% linezolid form II.

11. A pharmaceutical composition according to claim 10, where the polymorphically pure linezolid form III contains less than 10% linezolid form II.

12. A pharmaceutical composition according to claim 10, where the polymorphically pure linezolid form III contains less than 5% linezolid form II.

13. A pharmaceutical composition comprising polymorphically pure linezolid Form III and a pharmaceutically acceptable excipient wherein the composition is prepared by a method that avoids liquids or wet granulation using water as the granulating solvent.

14. The pharmaceutical composition of claim 13, wherein the linezolid Form III is polymorphically stable linezolid Form III.

15. A pharmaceutical composition comprising linezolid Form III and a pharmaceutically acceptable excipient, wherein the pharmaceutically acceptable excipient is an excipient that limits the conversion of linezolid Form III to Form II.

16. The pharmaceutical composition of claim 15, wherein the pharmaceutical composition contains no more than 20% linezolid Form II.

17. The pharmaceutical composition of claim 15, wherein the excipient is povidone.

18. The pharmaceutical composition of claim 15, wherein the pharmaceutical composition is free of mannitol.

19. A method of manufacturing a pharmaceutical composition according to any preceding claim, comprising wet granulating linezolid Form III with a pharmaceutically acceptable excipient using a granulation solvent selected from the group consisting of water, isopropanol, ethanol in the presence of povidone and water admixed with isopropanol or water admixed with ethanol in the presence of povidone.

20. The method of claim 19, wherein the linezolid Form III contains no more than 20% linezolid Form II.

21. The method of claim 19, wherein the granulating solvent selected from the group consisting of water and isopropanol.

22. The method of claim 19, wherein the pharmaceutically acceptable excipient comprises povidone.

23. A method of claim 19 wherein the granulating solvent is selected from the group consisting of water, isopropanol, or ethanol in the presence of povidone.

24. The method of claim 23, wherein the linezolid Form III contains no more than 20% linezolid Form U.

25. The method of claim 23, wherein the granulating solvent comprises water free of ethanol.

26. The method of claim 23, wherein the granulating solvent comprises isopropanol free of ethanol.

27. The method of claim 23, wherein the granulating solvent comprises ethanol in the presence of povidone.

28. A method of manufacturing a pharmaceutical composition according to any preceding claim, comprising dry granulating linezolid form III with a pharmaceutically acceptable excipient.

29. The method of claim 28, wherein the linezolid Form III contains no more than 20% linezolid Form II.

30. The method of claim 28, wherein the pharmaceutically acceptable excipient comprises povidone.

31. A method of manufacturing a pharmaceutical composition according to any preceding claim, comprising admixing linezolid form III with a pharmaceutically acceptable excipient to provide a mixture and direct compressing the mixture.

32. The method of claim 31, wherein the linezolid Form III contains no more than 20% linezolid Form II.

33. The method of claim 31, wherein the pharmaceutically acceptable excipient comprises povidone.

34. A method of limiting the conversion of linezolid Form III to linezolid Form II during formulation of a pharmaceutical composition as defined in any preceding claim, comprising at least one of:
i. formulating the pharmaceutical composition by wet granulation using a granulating solvent that is free of ethanol;
ii. formulating the pharmaceutical composition by wet granulation using a granulating solvent, wherein during formulation the granulating solvent contacts the linezolid Form III in the presence of povidone;
iii. including povidone in the pharmaceutical composition; and
iv. keeping the pharmaceutical composition free of sugar alcohols.

35. The method of claim 34, wherein the method comprises formulating the pharmaceutical composition by wet granulation using a granulating solvent selected from the group consisting of water and isopropanol.

36. The method of claim 34, wherein the method comprises keeping the pharmaceutical composition free of sugar alcohols and the sugar alcohol is mannitol.

37. The method or composition of any preceding claim, wherein the composition is or the method comprises keeping the pharmaceutical composition free of sugar alcohols and the sugar alcohol is mannitol.

## Patentansprüche

1. Pharmazeutische Zusammensetzung, welche hinsichtlich Polymorphie stabiles Linezolid Form III und einen pharmazeutisch verträglichen Hilfsstoff umfaßt, **dadurch gekennzeichnet, daß** die pharmazeutische Zusammensetzung nicht mehr als 10% Umwandlung von Linezolid Form III in Linezolid Form II zeigt, wenn sie bei 25°C/60% relativer Feuchte für 3 Monate gelagert wird, wobei die Zusammensetzung durch ein Verfahren hergestellt wird, welches Flüssigkeiten vermeidet, oder durch Naßgranulation unter Verwendung eines Granulationslösungsmittels, ausgewählt aus der Gruppe, bestehend aus Wasser, Isopropanol und Ethanol, in der Gegenwart von Povidon und Wasser im Gemisch mit Isopropanol oder Wasser im Gemisch mit Ethanol in der Gegenwart von Povidon.

2. Pharmazeutische Zusammensetzung nach Anspruch 1, wobei der Hilfsstoff Povidon ist.

3. Pharmazeutische Zusammensetzung nach Anspruch 1, wobei die pharmazeutische Zusammensetzung frei von Mannitol ist.

4. Pharmazeutische Zusammensetzung nach Anspruch 1, wobei die pharmazeutische Zusammensetzung ein Feststoff ist.

5. Pharmazeutische Zusammensetzung nach Anspruch 4, wobei die pharmazeutische Zusammensetzung in Form einer Tablette oder einer Kapsel vorliegt.

6. Pharmazeutische Zusammensetzung nach Anspruch 1, wobei die pharmazeutische Zusammensetzung weniger als 30 Prozent Umwandlung von Linezolid Form III in Linezolid Form II zeigt, wenn die pharmazeutische Zusammensetzung für 3 Monate bei 40°C und 75% relativer Feuchte gehalten wird.

7. Pharmazeutische Zusammensetzung nach Anspruch 1, wobei die pharmazeutische Zusammensetzung weniger als 25 Prozent Umwandlung von Linezolid Form III in Linezolid Form II zeigt, wenn die pharmazeutische Zusammensetzung für 2 Monate bei 40°C und 75% relativer Feuchte gehalten wird.

8. Pharmazeutische Zusammensetzung nach Anspruch 1, wobei die pharmazeutische Zusammensetzung weniger als 20 Prozent Umwandlung von Linezolid Form III in Linezolid Form II zeigt, wenn die pharmazeutische Zusammensetzung für 1 Monat bei 40°C und 75% relativer Feuchte gehalten wird.

9. Pharmazeutische Zusammensetzung nach einem der vorangegangenen Ansprüche, wobei das hinsichtlich Polymorphie stabile Linezolid Form III hinsichtlich Polymorphie reines Linezolid Form III ist, und ein pharmazeutisch verträglicher Hilfsstoff.

10. Pharmazeutische Zusammensetzung nach einem der vorangegangenen Ansprüche, wobei das hinsichtlich Polymorphie reine Linezolid Form III weniger als 20% an Linezolid Form II enthält.

11. Pharmazeutische Zusammensetzung nach Anspruch 10, wobei das hinsichtlich Polymorphie reine Linezolid Form III weniger als 10% an Linezolid Form II enthält.

12. Pharmazeutische Zusammensetzung nach Anspruch 10, wobei das hinsichtlich Polymorphie reine Linezolid Form III weniger als 5% an Linezolid Form II enthält.

13. Pharmazeutische Zusammensetzung, welche hinsichtlich Polymorphie reines Linezolid Form III und einen pharmazeutisch verträglichen Hilfsstoff umfaßt, wobei die Zusammensetzung durch ein Verfahren hergestellt wird, welches Flüssigkeiten vermeidet, oder durch Naßgranulation unter Verwendung von Wasser als Granulationslösungsmittel.

14. Pharmazeutische Zusammensetzung nach Anspruch 13, wobei das Linezolid Form III hinsichtlich Polymorphie stabiles Linezolid Form III ist.

15. Pharmazeutische Zusammensetzung, welche Linezolid Form III und einen pharmazeutisch verträglichen Hilfsstoff umfaßt, wobei der pharmazeutisch verträgliche Hilfsstoff ein Hilfsstoff ist, welcher die Umwandlung von Linezolid Form III in Form III beschränkt.

16. Pharmazeutische Zusammensetzung nach Anspruch 15, wobei die pharmazeutische Zusammensetzung nicht mehr als 20% an Linezolid Form II enthält.

17. Pharmazeutische Zusammensetzung nach Anspruch 15, wobei der Hilfsstoff Povidon ist.

18. Pharmazeutische Zusammensetzung nach Anspruch 15, wobei die pharmazeutische Zusammensetzung frei von Mannitol ist.

19. Verfahren zur Herstellung einer pharmazeutischen zusammensetzung nach einem der vorangegangenen Ansprüche, welches das Naßgranulieren von Form III mit einem pharmazeutisch verträglichen Hilfsstoff unter Verwendung eines Granulationslösungsmittels, ausgewählt aus der Gruppe, bestehend aus Wasser, Isopropanol und Ethanol in der Gegenwart von Povidon und Wasser im Gemisch mit Isopropanol oder Wasser im Gemisch mit Ethanol in der Gegenwart von Povidon, umfaßt.

20. Verfahren nach Anspruch 19, wobei das Linezolid Form III nicht mehr als 20% an Linezolid Form II enthält.

21. Verfahren nach Anspruch 19, wobei das Granulationslösungsmittel aus der Gruppe ausgewählt ist, bestehend aus Wasser und Isopropanol.

22. Verfahren nach Anspruch 19, wobei der pharmazeutisch verträgliche Hilfsstoff Povidon umfaßt.

23. Verfahren nach Anspruch 19, wobei das Granulationslösungsmittel aus der Gruppe ausgewählt ist, bestehend aus Wasser, Isopropanol oder Ethanol in der Gegenwart von Povidon.

24. Verfahren nach Anspruch 23, wobei das Linezolid Form III nicht mehr als 20% an Linezolid Form II enthält.

25. Verfahren nach Anspruch 23, wobei das Granulationslösungsmittel Wasser enthält, welches frei von Ethanol ist.

26. Verfahren nach Anspruch 23, wobei das Granulationslösungsmittel Isopropanol enthält, welches frei von Ethanol ist.

27. Verfahren nach Anspruch 23, wobei das Granulationslösungsmittel Ethanol in der Gegenwart von Povidon umfaßt.

28. Verfahren zur Herstellung einer pharmazeutischen Zusammensetzung nach einem der vorangegangenen Ansprüche, welches das Trockengranulieren von Linezolid Form III mit einem pharmazeutisch verträglichen Hilfsstoff umfaßt.

29. Verfahren nach Anspruch 28, wobei das Linezolid Form III nicht mehr als 20% an Linezolid Form II enthält.

30. Verfahren nach Anspruch 28, wobei der pharmazeutisch verträgliche Hilfsstoff Povidon umfaßt.

31. Verfahren zur Herstellung einer pharmazeutischen Zusammensetzung nach einem der vorangegangenen Ansprüche, welches das Mischen von Linezolid Form III mit einem pharmazeutisch verträglichen Hilfsstoff unter Bereitstellung eines Gemischs und das direkte Komprimieren des Gemischs umfaßt.

32. Verfahren nach Anspruch 31, wobei das Linezolid Form III nicht mehr als 20% an Linezolid Form II enthält.

33. Verfahren nach Anspruch 31, wobei der pharmazeutisch verträgliche Hilfsstoff Povidon umfaßt.

34. Verfahren zum Beschränken der Umwandlung von Linezolid Form III in Linezolid Form II während der Formulierung einer pharmazeutischen Zusammensetzung, wie sie in einem der vorangegangenen Ansprüche definiert ist, wobei das Verfahren wenigstens eines der folgenden umfaßt:
i. Formulieren der pharmazeutischen Zusammensetzung durch Naßgranulation unter Verwendung eines Granulationslösungsmittels, weiches frei von Ethanol ist,
ii. Formulieren der pharmazeutischen zusammensetzung durch Naßgranulation unter Verwendung eines Granulationslösungsmittels, wobei während der Formulierung das Granulationslösungsmittel mit dem Linezolid Form III in der Gegenwart von Povidon in Kontakt kommt,
iii. Aufnehmen von Povidon in die pharmazeutische Zusammensetzung und
iv. Halten der pharmazeutischen Zusammensetzung frei von Zuckeralkoholen.

35. Verfahren nach Anspruch 34, wobei das Verfahren das Formulieren der pharmazeutischen Zusammensetzung durch Naßgranulation unter Verwendung eines Granulationslösungsmittels, ausgewählt aus der Gruppe, bestehend aus Wasser und Isopropanol, umfaßt.

36. Verfahren nach Anspruch 34, wobei das Verfahren das Halten der pharmazeutischen Zusammensetzung frei von Zuckeralkoholen umfaßt und der Zuckeralkohol Mannitol ist.

37. Verfahren oder Zusammensetzung nach einem der vorangegangenen Ansprüche, wobei die Zusammensetzung frei von Zuckeralkoholen ist oder das Verfahren das Halten der pharmazeutischen Zusammensetzung frei von Zuckeralkoholen umfaßt und der Zuckeralkohol Mannitol ist.

## Revendications

1. Composition pharmaceutique comprenant la forme III du linézolide polymorphiquement stable et un excipient pharmaceutiquetnent acceptable, **caractérisée en ce que** la composition pharmaceutique ne présente pas plus de 10 % de conversion de la forme III du linézolide en forme II du linézolide lorsqu'elle est stockée à 25 °C/60 % d'humidité relative (HR) pendant 3 mois, ladite composition étant préparée par un procédé qui évite les liquides ou une granulation humide utilisant un solvant de granulation choisi dans le groupe constitué par l'eau, l'isopropanol, l'éthanol en présence de povidone et l'eau mélangée avec de l'isopropanol ou l'eau mélangée avec de l'éthanol en présence de povidone.

2. Composition pharmaceutique selon la revendication 1, dans laquelle l'excipient est la povidone.

3. Composition pharmaceutique selon la revendication 1, dans laquelle la composition pharmaceutique est exempte de maunitol.

4. Composition pharmaceutique selon la revendication 1, dans laquelle la composition pharmaceutique est un solide,

5. Composition pharmaceutique selon la revendication 4, dans laquelle la composition pharmaceutique est sous la forme d'un comprimé ou d'une gélule.

6. Composition pharmaceutique selon la revendication 1, dans laquelle la composition pharmaceutique présente moins de 30 % de conversion de la forme III du linézolide en forme II du linézolide lorsque la composition pharmaceutique est maintenue à 40°C et 75 % d'humidité relative pendant 3 mois.

7. Composition pharmaceutique selon la revendication 1, dans laquelle la composition pharmaceutique présente moins de 25 % de conversion de la forme III du linézolide en forme II du linézolide lorsque la composition pharmaceutique est maintenue à 40 °C et 75 % d'humidité relative pendant 2 mois.

8. Composition pharmaceutique selon la revendication 1, dans laquelle la composition pharmaceutique présente moins de 20 % de conversion de la forme III du linézolide en forme II du linézolide lorsque la composition pharmaceutique est maintenue à 40 °C et 75 % d'humidité relative pendant 1 mois.

9. Composition pharmaceutique selon l'une quelconque des revendications précédentes, dans laquelle la forme III du linézolide polymorphiquement stable est la forme III du linézolide polymorphiquement pure et un excipient pharmaceutiquement acceptable.

10. Composition pharmaceutique selon l'une quelconque des revendications précédentes, dans laquelle la forme III du linézolide polymorphiquement pure contient moins de 20 % de la forme II du linézolide,

11. Composition pharmaceutique selon la revendication 10, dans laquelle la forme III du iinèzolide polymorphiquement pure contient moins de 10 % de la forme II du linézolide.

12. Composition pharmaceutique selon la revendication 10, dans laquelle la forme III du linézolide polymorphiquement pure contient moins de 5 % de la forma II du linézolide.

13. Composition pharmaceutique comprenant la forme III du linézolide polymorphiquement pure et un excipient pharmaceutiquement acceptable, dans laquelle la composition est préparée par un procédé qui évite les liquides ou une granulation humide utilisant de l'eau en tant que solvant de granulation.

14. Composition pharmaceutique selon la revendication 13, dans laquelle la forme III du linézolide est la forme III du linézolide polymorphiquement stable.

15. Composition pharmaceutique comprenant la forme III du linézolide et un excipient pharmaceutiquement acceptable, dans laquelle l'excipient pharmaceutiquement acceptable est un excipient qui limite la conversion de la forme III du linézolide en forme II.

16. Composition pharmaceutique selon la revendication 15, dans laquelle la composition pharmaceutique ne contient pas plus de 20 % de la forme II du linézolide.

17. Composition pharmaceutique selon la revendication 15, dans laquelle l'excipient est la povidone.

18. Composition pharmaceutique selon la revendication 15, dans laquelle la composition pharmaceutique est exempte de mannitol.

19. Procédé de fabrication d'une composition pharmaceutique selon l'une quelconque des revendications précédentes, comprenant la granulation humide de la forme III du linézolide avec un excipient pharmaceutiquement acceptable utilisant un solvant de granulation choisi dans le groupe constitué par l'eau, l'isopropanol, l'éthanol en présence de povidone et d'eau mélangé avec de l'isopropanol ou l'eau mélangée avec de l'éthanol en présence de povidone.

20. Procédé selon la revendication 19, dans lequel la forme III du linézolide ne contient pas plus de 20 % de la forme II du linézolide.

21. Procédé selon la revendication 19, dans lequel le solvant de granulation est choisi dans le groupe constitué par l'eau et l'isopropanol.

22. Procédé selon la revendication 19, dans lequel l'excipient pharmaceutiquement acceptable comprend la povidone.

23. Procédé selon la revendication 19, dans lequel le solvant de granulation est choisi dans le groupe constitué par l'eau, l'isopropanol ou l'éthanol en présence de povidone.

24. Procédé selon la revendication 23, dans lequel la forme III du linézolide ne contient pas plus de 20 % de la forme II du linézolide.

25. Procédé selon la revendication 23, dans lequel le solvant de granulation comprend de l'eau sans éthanol.

26. Procédé selon la revendication 23, dans lequel le solvant de granulation comprend de l'isopropanol sans éthanol.

27. Procédé selon la revendication 23, dans lequel le solvant de granulation comprend de l'éthanol en présence de povidone.

28. Procédé de fabrication d'une composition pharmaceutique selon l'une quelconque des revendications précédentes, comprenant la granulation sèche de la forme III du linézolide avec un excipient pharmaceutiquement acceptable.

29. Procédé selon la revendication 28, dans lequel la forme III du linézolide ne contient pas plus de 20 % de la forme II du linézolide.

30. Procédé selon la revendication 28, dans lequel l'excipient pharmaceutiquement acceptable comprend la povidone.

31. Procédé de fabrication d'une composition pharmaceutique selon l'une quelconque des revendications précédentes, comprenant le mélange de la forme III du linézolide avec un excipient pharmaceutiquement acceptable pour obtenir un mélange et la compression directe du mélange.

32. Procédé selon la revendication 31, dans lequel la forme III du linézolide ne contient pas plus de 20 % de la forme II du linézolide.

33. Procédé selon la revendication 31, dans lequel l'excipient pharmaceutiquement acceptable comprend la povidone.

34. Procédé de limitation de la conversion de la forme III du linézolide en forme II du linézolide pendant la formulation d'unc composition pharmaceutique selon l'une quelconque des revendications précédentes, comprenant au moins une des étapes de :
i. formulation de la composition pharmaceutique par granulation humide en utilisant un solvant de granulation exempt d'éthanol ;
ii. formulation de la composition pharmaceutique par granulation humide en utilisant un solvant de granulation, où pendant la formulation le solvant de granulation entre en contact avec la forme III du linézolide en présence de povidone ;
iii. inclusion de la povidone dans la composition pharmaceutique ; et
iv. conservation de la composition pharmaceutique exempte de polyols.

35. Procédé selon la revendication 34, dans lequel le procédé comprend la formulation de la composition pharmaceutique par granulation humide utilisant un solvant de granulation choisi dans le groupe constitué par l'eau et l'isopropanol.

36. Procédé selon la revendication 34, dans lequel le procédé comprend la conservation de la composition pharmaceutique exempte d'alcools de polyols et le polyol est le mannitol.

37. Procédé ou composition selon l'une quelconque des revendications précédentes, dans lequel/laquelle la composition est ou le procédé comprend la conservation de la composition pharmaceutique exempte de polyols et le polyol est le mannitol.
